(19) Europäisches Patentamt
European Patent Office
Office européen des brevets

(11) Publication number: **0 145 447**
**A1**

(12) # EUROPEAN PATENT APPLICATION

(21) Application number: **84308450.0**

(51) Int. Cl.⁴: **C 07 D 301/02**

(22) Date of filing: **05.12.84**

(30) Priority: **08.12.83 US 559428**
**20.11.84 US 672271**

(43) Date of publication of application: **19.06.85**
**Bulletin 85/25**

(84) Designated Contracting States: **AT BE DE FR GB IT NL**

(71) Applicant: **Exxon Research and Engineering Company, P.O.Box 390 180 Park Avenue, Florham Park New Jersey 07932 (US)**

(72) Inventor: **Ryu, Ji-Yong, 196 Nottingham Road, Ramsey New Jersey (US)**

(74) Representative: **Dew, Melvyn John et al, Esso Chemical Ltd. Esso Chemical Research Centre P.O. Box 1, Abingdon Oxfordshire, OX13 6BB (GB)**

(54) **Process for epoxidizing vicinal diols.**

(57) Vicinal diols are epoxidised in the presence of a promoter comprising water, aldehyde or ketone using as catalyst the hydroxide, oxide or carboxylates of certain alkali metals supported on amorphous silica.

EP 0 145 447 A1

# PROCESS FOR EPOXIDIZING VICINAL DIOLS

The present invention relates to a process for epoxidizing vicinal diols such as propylene glycol with a two component catalyst composition in the presence of a promoter co-feed to improve diol conversion and/or epoxide selectivity.

The epoxides constitute a class of compounds of which the industrial importance is measured by the tonnages produced and by the diversity of their applications in the field of urethanes, glycols, surface-active agents, plasticizers and numerous other derivatives.

While many specific methods for producing epoxides are known, the most prominent of these methods use olefins as a starting material and can generally be divided into four basic types. For example, the oldest industrial technique for the epoxidation of double bonds is the process known as chlorohydrin process. In the chlorohydrin process an olefin is reacted with chlorine in an alkaline medium. The yields (based on the chlorine) are unsatisfactory. This process also gives rise to the simultaneous formation of considerable quantities of chlorinated by-products, both inorganic and organic, which products are unsuitable for any known purpose. The disposal of these by-products involves problems of such magnitude that this process may eventually be abandoned.

The second type of epoxidation method is generally limited to the epoxidation of ethylene. In accordance with this method ethylene is epoxidized with good yields in the vapor phase by means of molecular oxygen over a catalyst on a silver base. However, this technique is not very useful for the higher carbon olefins because of its lack of selectivity.

A third and more recent type of epoxidation process is characterized by the use of organic hydroperoxides. In accordance with processes of this type, an olefin is epoxidized catalytically in an organic medium containing an organic hydroperoxide oxidant. In addition to employing a

relatively expensive organic hydroperoxide as an oxidant, it is also a characteristic disadvantage of these processes that the epoxide formation is accompanied by the formation in an equivalent or even greater amount of an alcohol derived from the organic hydroperoxide employed in the process. Consequently, the commercial viability of these processes is always influenced by the ability to economically dispose of the alcohol by-product in addition to the epoxide.

A fourth type of olefin epoxidation process is characterized by the use of hydrogen peroxide as the oxidant. Hydrogen peroxide is in principle a desirable reagent, for the very reason of its oxidizing non-polluting nature. However, its reactivity towards olefins is weak or non-existent in the absence of an activating agent which enables a more active peroxy compound to be formed in-situ. Different processes of epoxidation have therefore been proposed using, for example, organic per-acids such as performic, peracetic or perpropionic acids. Nevertheless, because of the instability of the epoxides in acid medium, such processes are particularly difficult to put into practice.

Accordingly, new alternative methods of access to epoxides are continually being sought.

It is acknowledged that glycols are often manufactured from epoxides by hydrolysis of an epoxide ring. Consequently, at first sight it might appear that the manufacture of epoxides from glycols is a circuitous approach.

However, recent developments in osmium based olefin hydroxylation systems such as illustrated in commonly assigned U.S. Patent Nos. 4,314,088; 4,393,253; and 4,390,739 have led to the direct hydroxylation of olefins to glycols at nearly quantitative yields with very little by-product formation. Consequently, glycols produced in accordance with these processes would provide an ideal inexpensive source of glycol feed while by-passing the disadvantages of the synthesis of epoxides directly from olefins.

French Patent No. 2,345,440, assigned to Rhone-Poulenc Ind., discloses a process for producing epoxides from alkane-1,2-diols in the liquid or vapor phase with a basic substance whose aqueous solutions thereof have a pH between 8 and 12 at a concentration of 0.1 mole/l. The basic materials disclosed are carboxylates, e.g., acetates, borated slats, ortho- and pyro-phosphates, aluminates, silicates, arsenates, and hydroxides of Groups I, II and III of the periodic chart, particularly sodium, potassium, lithium, calcium, and barium. (Note that except for the Group I, II or III hydroxides, such basic materials are salts derived by reacting a strong base with a weak acid). The most efficient basic materials are said to be potassium acetate, sodium tetraborate, sodium or lithium orthophosphate, and sodium meta- and di-silicate. Such materials can be supported, but it is specifically stated that supports having an acidic character should be avoided since the reaction would be directed to the preferential formation of aldehydes. Suitable disclosed supports include alpha-alumina, silicon carbide, and alkaline silica-aluminates.

Inert diluents can be included in the glycol feed such as nitrogen or helium, or a hydrocarbon such as benzene, toluene or xylene. The addition of water, aldehydes or ketones to the feed is not disclosed.

U.S. Patent No. 4,226,780 discloses the epoxidation of propylene glycol, or mixtures of propylene glycol and its mono- and/or di-esters, with a two component catalyst containing a weakly acidic component (e.g., carrier) and a basic component. The disclosed acidic components are titanium dioxide, zirconium dioxide, and especially silica. The basic component is an alkali metal compound applied to the acidic carrier component in an amount of 5 to 25% by weight. The only specific examples of suitable alkali metal compounds disclosed are carboxylates, such as acetates, and compounds which form carboxylates with carboxylic acids under epoxidation reaction conditions. Alkali metal hydroxides are not

disclosed as suitable basic components. Water and/or aldehydes or ketones are not disclosed as a co-feed for use in conjunction with vicinal diols.

U.S. Patent No. Re. 29,597 discloses a process for producing epoxides by the deacyloxylation of vicinal hydroxy esters in the presence of a basic material. Suitable disclosed basic materials are carboxylates of Group I, II and IIIA metals, particularly sodium, potassium, lithium, calcium and barium and compounds which form carboxylates in-situ, e.g., by reaction with acetic acid such as simple oxides, complex oxides, and organic bases of the aforedescribed metals. Additional disclosed basic materials disclosed include alkali and alkaline earth metal salts of boric acid. The above basic materials can be employed on a support. However, it is stated that acidic supports are not desirable because they favor aldehyde formation (col. 4, lines 14 et. seq.). The reaction sequence believed to be operating in the process of this patent is described as involving inter-alia, the reaction of the basic material with the carboxylate portion of the hydroxy ester to produce a carboxylate salt (e.g., sodium acetate) that is removed from the reaction zone by distillation as, for example, acetic acid. The hydroxy esters may be fed to the reaction zone alone or if desired, diluted with a carrier gas. The carrier is disclosed to act as a heat sink and to lower the partial pressure of the hydroxy ester in the reaction. The carrier may be a liquid, i.e. condensable at room temperature, such as benzene, toluene, xylene, pseudocumene, and water. Regarding the last carrier, i.e. water, it will be noted that the formation of the epoxide from the hydroxy ester does not form water, in contrast to the dehydration of diols which does. Consequently, one would not expect water to have an adverse influence on epoxide selectivity and/or conversion in the former reaction by shifting the equilibrium away from product formation.

U.S. Patent No. 4,012,424 is directed to a process for cracking feed mixtures of hydroxy esters, diesters and glycols to form epoxides in the presence of a basic catalyst. The feed so cracked must contain at least 10% by weight hydroxy ester when a carrier gas is employed. Suitable basic catalysts disclosed are alkali or alkaline earth metal borates, phosphates, oxides, carbonates, aluminates or silicates. When these materials are supported, however, the support must be neutral or basic such as alpha-alumina, silicon carbide, silicon silicate, zirconium silicate and aluminum silicate. Suitable carriers are the same as disclosed in Re. 29,597.

U.S. Patent No. 4,399,295 is directed to a process for the vapor phase deacyloxylation of vicinal hydroxy esters utilizing a dilute phase transport reactor as the reaction zone and a fludized bed for heating and reactivating the catalyst. The feed employed can be as disclosed in U.S. Patent No. 4,012,424. It is an advantage of this reactor design that steam can be employed for catalyst regeneration, in a separate zone and removed before feeding the catalyst to the reaction zone thereby avoiding hydrolysis of the product and the associated reduction in selectivity (col. 5, lines 15 et. seq.).

The formation of silica gel supported alkali metal hydroxides is disclosed in U.S. Patent No. 3,070,639. However, such supported hydroxides are employed as an adsorbent for polar organic compounds. See also U.S. Patent Nos. 3,840,588; 3,933,888; and 4,229,320 for the use of silica supported hydroxides in environments totally different than the process of the present invention.

Thus, there has been a continuing search for alternative and improved ways to produce epoxides from polyols and the present invention was developed in response to this search.

The process of the present invention relies on the use of a specific catalyst composition to epoxidize vicinal polyols in conjunction with a specifically defined promoter which is included in the polyol feed to enhance selectivity and/or conversion.

Accordingly, in one aspect of the present invention there is provided a process for directly epoxidizing at least one organic polyol compound containing at least one pair of vicinal hydroxy groups which comprises:

(a) providing a feed consisting essentially of a mixture of said polyol compound and at least one promoter selected from the group consisting of water, aldehyde or ketone; said promoter having an identity and being present in an amount sufficient to increase at least one of the conversion of said polyol and selectivity to epoxide, relative to the absence of said promoter;

(b) contacting said feed with a catalyst composition comprising a solid acidic amorphous silica having adsorbed thereon, at least one member selected from the group consisting of alkali metal hydroxide, alkali metal oxide, and alkali metal carboxylate, said alkali metal being selected from at least one member of the group consisting of K, Rb, and Cs, said contact being conducted in a manner and under conditions sufficient to convert at least one pair of said vicinal hydroxy groups to its corresponding epoxide group.

The present invention employs a two component catalyst composition comprising as a first component, a weakly acidic, amorphous silica support, impregnated, e.g., by adsorption, with a second catalyst component comprising at least one alkali metal hydroxide, oxide, or carboxylate, said metal being selected from the group consisting of K, Rb, and

Cs, preferably Rb and Cs, as an epoxidation catalyst for vicinal diols. The Li or Na oxides or hydroxides provide ineffective catalysts.

More specifically, the amorphous silica employed in the present invention is weakly acidic and is conventional. The preferred form of amorphous silica is silica gel, which contains coherent, rigid, continuous three-dimensional networks of aggregated silica particles of colloidal dimensions. Silica gels suitable for use herein include the regular, intermediate, and low density types, but are preferably of intermediate density.

More specifically, regular density silica gel is made by gelling in an acid medium, which gives very small particles with high surface area (e.g., 750 - 800 $m^2/g$). The average pore diameter is about 2.2 - 2.6 nm, and the pore volume is about 0.37 - 0.40 cc/g. Typically, regular density gel contains about 6 wt.% water as surface hydroxyl groups, which imparts a high capacity for water adsorption and adsorption of other polar molecules. Regular density silica gel exhibits a high selectivity for polar molecules and a large percentage of small pores.

Intermediate-density silica gel has a lower surface area (e.g., 300 - 350 $m^2/g$) but larger pore volume (e.g., 0.9 - 1.1 cc/g). The average pore size is about 12- 16 nm in diameter, and the particles are larger than those of regular-density silica gel. Because of the large pore size, intermediate-density silica gel has a high capacity for water adsorption at high humidities.

Low density silica gel has a lower surface area (e.g., 100 - 200 $m^2/g$), larger average pore diameter (e.g., 18 - 22 nm), and larger pore volume (e.g., 1.4 -2.0 cc/g) than the other types. It is usually prepared as a very fine powder of extremely low density; shrinkage of the gel during drying is minimized.

Other forms of amorphous silica suitable for use herein include colloidal silica, precipitated silica, and the like.

The second catalyst component which is adsorbed by the amorphous silica is basic to the latter and comprises hydroxides, oxides and/or carboxylates of the aforedescribed alkali metals. Such materials are incorporated into the first catalyst component by physical and/or chemical adsorption which results in intimate and impregnation into the first component.

When an appropriate alkali metal carboxylate is employed for impregnation of the silica support, the anion thereof can be represented by structural formula:

$$(R_1)-\overset{O}{\overset{\|}{C}}-O^- \qquad (I)$$

wherein $R_1$ is selected from the group consisting of alkyl, typically $C_1$ to $C_{10}$ alkyl, preferably $C_1$ to $C_5$ alkyl, and most preferably $C_1$ to $C_3$ alkyl, aryl, typically $C_6$ to $C_{10}$ aryl, aralkyl and alkaryl wherein the alkyl and aryl portions thereof are as described above.

Representative carboxylate anions include acetate, propanoate, butanoate, benzoate and mixtures thereof, the preferred carboxylate is the acetate.

The amount of the second catalyst component adsorbed on the amorphous silica will typically be not less than 3%, preferably not less than 4% and most preferably not less than 5%, typically from 3 to 35, preferable from 4 to 20, and most preferably from 5 to 15%, by weight, based on the combined weight of dried catalyst composition.

If a potassium compound is employed as the modifying agent (i.e. second catalyst component), it is typically present on the silica gel in amounts of from 3 to 15% (e.g., 4 to 12%), by weight, based on the weight of the catalyst composition.

If a rubidium compound is employed as the modifying agent, it is typically present on the silica gel in amounts of from 4 to 25% (e.g., 6 to 20%), by weight based on the weight of the catalyst composition.

If a cesium compound is employed as the modifying agent, it is typically present on the silica gel in amounts of from 7 to 35% (e.g., 9 to 35%), by weight based on the weight of the catalyst composition.

The use of a cesium hydroxide supported catalyst composition is preferred.

The second catalyst component is adsorbed on the first catalyst component by any way capable of achieving physical and/or chemical adsorption of the latter by the silica support. Thus, adsorption methods are used which result in retention of the second catalyst component in or on the silica support, and various materials and procedures are suitable for this purpose.

Thus, for example, the second catalyst component can conveniently be impregnated into the pores and surface of the silica support by utilizing a solution, preferably an aqueous solution of the same as a slurrying medium for the silica support particles. Ammonia or other base can be added to the mixture to cause and/or facilitate precipitation of the second catalyst component on the silica support.

Solvents other than water (e.g. methanol) can also be employed as a suspending medium and/or solvent for catalyst components I and II.

Impregnation temperatures at which the aqueous solution of second catalyst component are admixed with the first component typically can be from 10 to 200, and preferably from 15 to 100°C.

Impregnation pressures are not critical and can be atmospheric, subatmospheric, or superatmospheric.

Catalyst components I and II typically are allowed to remain in contact in solution for a period sufficient to achieve adsorption in amounts as described above. Such adsorption times typically vary from 0.1 to 10, and preferably from 0.1 to 4 hours.

The product resulting from impregnation is then typically separated from the liquid mixing medium, e.g., water, by any suitable technique such as by filtration, centrifuging, decanting and/or evaporation at atmospheric or reduced pressure.

The recovered wet product is then typically dried.

Drying can be achieved by exposing the impregnation product to air or inert gas at a temperature of from 20 to 200°C, for a period of from 0.1 to 40 hours until constant weight is attained.

When oxides or hydroxides are employed as the modifying agent, it is preferred to cause such second catalyst component to react, e.g., with the hydroxyl groups of the silica gel on which it is adsorbed, the dried catalyst compostition is preferably heated, i.e., calcined to achieve such reaction.

Calcination can be conducted in a separate step or in-situ in the reactor and involves heating the catalyst composition to a selected temperature or temperatures within a defined temperature range. Preferably the calcination procedure is conducted by heating the catalyst, preferably in an inert gas such as nitrogen, at a temperature of typically from 200 to 950, and preferably from 250 to 500°C, for a period of typically from 0.1 to 40, preferably from 0.1 to 15 hours.

The material which is epoxidized in accordance with the process of the present invention is an organic polyol containing at least one pair of vicinal hydroxy groups. Thus, while the polyol may contain two or more hydroxy groups, only at least two of these hydroxy groups must be located on adjacent carbons, and hence are characterized as vicinal hydroxy groups. Furthermore, the polyol may contain two or more vicinal diol groupings. The polyol typically will have from 2 to 34, preferably from 3 to 20 and most preferably from 3 to 10 carbon atoms. If the polyol is to be epoxidized in the vapor phase, its identity is selected to permit vaporization thereof at a combination of temperature and pressure below which its decomposes.

Suitable polyol reactants for use herein when noncyclic can be straight or branched chain. They can be aliphatic, aromatic, or cycloaliphatic, preferably saturated aliphatic.

The preferred polyols are alkane polyols, most preferably alkane vicinal diols typically having from 2 to 20 carbons and most preferably 3 to 10 carbons.

More specifically, organic polyols which may be directly epoxidized and the epoxidized product produced thereby may be represented by the following equation:

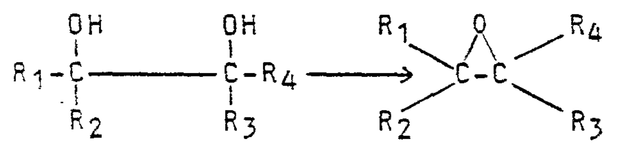 $+H_2O$ (Eq. 1)

wherein $R_1$, $R_2$, $R_3$ and $R_4$ which may be the same or different independently represent a member selected from the group consisting of hydrogen, alkyl, typically $C_1$ to $C_{20}$ alkyl, preferably $C_1$ to $C_{10}$ alkyl, most preferably $C_1$ to $C_5$ alkyl, aryl, typically $C_6$ to $C_{14}$ aryl, preferably $C_6$ to $C_{10}$ aryl, and most preferably $C_6$ aryl, alkenyl, typically $C_2$ to $C_{20}$ alkenyl, preferably $C_2$ to $C_{10}$ alkenyl, and most preferably $C_2$ to $C_6$, alkenyl, alkaryl and aralkyl wherein the alkyl and aryl portions thereof are as described above. Furthermore, any two of said $R_1$ to $R_4$ groups together can constitute a cycloalkylene group, typically $C_4$ to $C_{16}$ cycloalkylene, preferably $C_5$ to $C_{12}$ cycloalkylene, and most preferably $C_6$

to $C_{10}$ cycloalkylene. In addition the hydrocarbyl groups constituting $R_1$ to $R_4$ may be substituted with one or more hydroxy substituents.

Preferably at least two of said $R_1$ to $R_4$ groups are hydrogen and the remainder of said R groups being independently methyl, ethyl, propyl, butyl or phenyl.

Representative examples of suitable vicinal diols include ethylene glycol, propylene glycol, 1,2-cyclopentanediol, 1,2-butanediol, 2-methyl-1,2-propanediol, 1,2-cyclohexanediol, 1,2-dihydroxybenzonitrile, 1,2,3,4-tetrahydro-2,3-naphthalenediol, glycerol and mixtures thereof.

The most preferred glycols are ethylene glycol, and propylene glycol.

To avoid loss of the alkali metal of the catalyst during reaction, it is preferred to exclude the presence of a carboxylic acid from the reactant feed and conduct the epoxidation reaction in the absence of the same. Small amounts of such acids in the feed can be tolerated however.

While the epoxidation reaction can be conducted in the liquid phase using a liquid polyol, the epoxidation reaction is preferably conducted in the vapor phase in the presence of the catalyst described hereinabove, continuously or batchwise, in a fixed or fluidized bed.

However, it is critical to the present invention that a promoter be included in the polyol feed. The promoter is of two general classes, namely (i) water and (ii) a carbonyl containing compound, i.e. aldehydes or ketones. Each class of promoters can be employed alone or in combination. The carbonyl containing promoter preferably should be vaporizable at reaction temperatures.

The effect of the promoter is to increase conversion of the polyol and/or selectivity of the reaction to epoxide.

Regarding the use of water as a promoter, it will be observed that the dehydration of vicinal diols to epoxides produces one mole of water per mole of epoxide. In contrast,

the use of hydroxy esters as the feed does not produce water, but an acid instead. Consequently, one would expect that the addition of water to a feed consisting essentially of vicinal diol, e.g. in the absence of hydroxy ester in the feed, would be unfavorable for the formation of epoxide product. This unexpectedly does not happen and in fact, the use of water in the feed enhances selectivity and/or conversion relative to its absence at similar on stream times.

Regarding the use of a carbonyl containing compound (i.e. ketone or aldehyde) as a promoter, and without wishing to be bound by any particular theory or mechanism, it is believed that the enhancement in selectivity and/or yield associated with the use of a ketone illustrated hereinafter is attributable to the carbonyl functional group present thereon. This class of promoters therefore contain one or more (e.g., 2) carbonyl groups.

Accordingly, suitable promoters which possess this active functional group may be represented by the structural formula:

$$R_5 - \overset{\overset{O}{\|}}{C} - R_6 \qquad (II)$$

wherein $R_5$ is alkyl, typically $C_1$ to $C_{10}$ alkyl, preferably $C_1$ to $C_5$ alkyl, and most preferably $C_1$ to $C_3$ alkyl; alkenyl, typically $C_2$ to $C_{10}$ alkenyl, preferably $C_2$ to $C_5$ alkenyl; aryl, typically $C_6$ to $C_{10}$ aryl, preferably $C_6$ aryl, aralkyl and alkaryl wherein the alkyl and aryl portions thereof are as described above, the alkyl, alkenyl, or alkyl portions of $R_5$ being optionally substituted with at least one oxo (O=) group; and $R_6$ is hydrogen or a hydrocarbyl group selected from alkyl, alkenyl, aryl, aralkyl and alkaryl as described in conjunction with $R_5$ including oxo substituents. Thus, when $R_6$ is hydrogen, the promoter (Structure II) is an aldehyde; and when $R_6$ is a hydrocarbyl group the promoter is a ketone.

Moreover, when $R_6$ is a hydrocarbyl group, $R_5$ and $R_6$ together can constitute a cycloalkylene group, typically $C_4$ to $C_{12}$ cycloalkylene, preferably $C_5$ to $C_8$ cycloalkylene, said cycloalkylene being also optionally substituted with at least one oxo group.

It is further believed that the reaction route involving such promoter is more selective and/or occurs at a faster rate than the direct dehydration of the vicinal diol. Consequently, the use of such carbonyl containing compounds is believed to provide a supplemental reaction route to the direct dehydration of vicinal diols, thereby enhancing the overal selectivity and/or conversion.

Accordingly representative ketone promoters include methylethylketone (MEK); 2-pentanone; 3-pentanone; 3-penten-2-one; 2,4-pentanedione; 2-hexanone; 3-hexanone; 2,5-hexanedione; acetone; 1,4-cyclohexanedione; cyclopentanone; diphenyl ketone; and mixtures thereof.

The preferred ketone is methylethylketone.

Representative aldehydes include formaldehyde; acetaldehyde; isobutylaldehyde; n-butyl aldehyde; propionaldehyde; 4-pentenal benzaldehyde; cyclohexane carbaldehyde; ethanedial; propane dial and mixtures thereof.

The preferred aldehyde is isobutylaldehyde.

While any amount of water promoter effective to increase the selectivity and/or conversion of the reaction relative to its absence can be employed, it is contemplated that such effective amount constitute from 0.1 to 25, preferably from 0.5 to 20, and most preferably from 0.8 to 15%, by weight, based on the weight of the feed mixture of vicinal diol and promoter.

Likewise, while any amount of the carbonyl containing promoter effective to increase the selectivity and/or conversion of the reaction relative to its absence can be employed, it is contemplated that such effective amount constitute from 1 to 95, preferably from 5 to 80, and most preferably from 10 to 75%, by

weight, based on the weight of the feed mixture of vicinal diol and promoter. For purposes of determining amounts of the carbonyl containing promoter herein, aldehydes are considered equivalent to ketones.

When a mixture of vicinal diol, water, and carbonyl containing promoter is employed the weight percent ratio of said components in the feed typically will vary from 50:1:1000 to 3:3:1, preferably from 10:1:40 to 5:4:2, and most preferably from 4:1:15 to 3:2:1.

Hydroxy ethers are intended to be excluded from the feed.

The catalyst may be charged to a tube, or on trays or in a fluidized bed, through which the feed mixture is passed. The reactor system may consist of a series of catalyst beds with optional interstage heating or cooling between the beds if desired. It is also an embodiment of the invention to use either upflow or downflow of the reactant feed through the reactor, with periodic reversal of gas flow also being contemplated to maintain a clean catalyst bed.

The feed mixture may be fed to the reaction zone alone or, if desired, diluted with an inert carrier gas. The carrier gas may be a liquid, i.e., condensable at room temperature, such as benzene, toluene, or xylenes. Noncondensable carrier gases may also be used. These include nitrogen, helium, and carbon dioxide. Generally, where a carrier is used, the carrier will typically comprise from 5 to 95, and preferably from 10 to 85%, by weight, based on the total gas volume of the feed at reaction temperature. The preferred carrier is nitrogen.

The manner of combining vicinal diol, promoter, and optional carrier is not critical provided the feed components contact the catalyst in vapor form when the process is conducted in the vapor phase.

The epoxidation reaction temperature will typically vary from 200 to 500, and preferably from 250 to 430°C.

The epoxidation reaction temperature typically will be selected from the aforenoted ranges in conjunction with the reaction pressure to assure contact of the reactant feed with the catalyst as a vapor.

A wide range of epoxidation pressures can be employed typically ranging from 0.1 to 50, and preferably from 0.5 to 35 psia. As a matter of convenience, atmospheric pressure is preferred. Where a carrier gas is employed, it is preferred that the partial pressure of the reactant diol be from 1 to 45, most preferably from 1.5 to 35 psia, and the partial pressure of the promoter be typically from 0.01 to 30, and preferably from 0.1 to 20 psia.

Suitable feed rates of reactant diol to the reaction zone typically can vary from 0.1 to 40, preferably from 0.2 to 30, and most preferably from 0.5 to 25 $hr.^{-1}$ LHSV.

The gas exiting from the reactor may be conveniently quenched in cooling coils or in a cold trap. The epoxide can be separated from the condensed carrier, if used, by distillation. Promoter can be recycled if desired.

The following examples are given as specific illustrations of the claimed invention. It should be understood, however, that the invention is not limited to the specific details set forth in the examples. All parts and percentages in the examples as well as in the remainder of the specification are by weight unless otherwise specified.

In the following examples, and comparative examples, unless otherwise specified, each catalyst was tested in the following manner: A glass tube reactor 20 inches in length, 1 inch O.D. and about 0.8 inch I.D., is stoppered at 15 inches from the top with glass wool, loaded with 5cc of catalyst sample on top of which is placed 20cc of glass wool,

followed by the addition of a sufficient number of 4mm diameter glass balls to fill the remaining reactor tube volume. The top eight inches of the reactor serves as a preheat zone for the feed. The reactor is mounted in a vertical furnace having a heating chamber 2.5cm in diameter and 30.5 cm in length. A liquid feed stream is then passed downward through the reactor tube at a selected furnace temperature as described herein. The feed stream is vaporized in the preheating zone and contacts the catalyst as a vapor. All reactions are carried out under ambient atmospheric pressure. The reactant feed stream is passed through the reactor at a liquid hourly space velocity (LHSV) as specified hereinafter. In some instances a nitrogen carrier gas is combined with the vaporized feed mixture. In other instances xylene is employed as an inert carrier gas which is admixed in liquid form with the reactant feed mixture.

Unless otherwise specified, conversion selectivity and yield are calculated as follows:

$$\text{PG conversion (\%)} = \frac{A-B}{A} \times 100$$

$$\text{Selectivity to PO (mole \%)} = \frac{C}{A-B} \times 100$$

$$\text{Selectivity to DPG (mole \%)} = \frac{G}{A-B} \times 100$$

$$\text{Selectivity to PA (\%)} = \frac{D}{A-B}$$

$$\text{Selectivity to Ac (\%)} = \frac{E}{A-B}$$

$$\text{Selectivity to AA (\%)} = \frac{F}{A-B}$$

$$\text{PO yield (\%)} = \frac{\text{selectivity} \times \text{conversion}}{100}$$

wherein: PG = propylene glycol
PO = propylene oxide
DPG = dipropylene glycol
Ac = acetone
AA = allyl alcohol
PA = propionaldehyde

A = moles of propylene glycol in the feed.
B = moles of propylene glycol in the reaction product.
C = moles of propylene oxide in the reaction product.
D = moles of propionaldehyde in the reaction product.
E = moles of acetone in the reaction product.
F = moles of allyl alcohol in the reaction product.
G = moles of dipropylene glycol in the reaction product.

Also, unless otherwise specified, the silica gel employed in the following examples and comparative examples is obtainable from Ventron Company, is characterized by a surface area of 300 $m^2/g$, a pore volume of lcc/g and a particle size of -9 + 16 mesh (Tyler series).

COMPARATIVE EXAMPLE 1

PART A

Silica gel (47.49g) granules were mixed with a CsOH solution prepared by dissolving 10.25g of CsOH in 300cc of water, in a vacuum rotary drier maintained at 62°C with a water bath for 2 hours.

The water was removed by evaporation at 90°C (water bath temperature) by applying vacuum to the rotary drier.

The dried catalyst was then placed in the reactor and calcined at 438°C for 2 hours, and 220° for 18 hours, while dry nitrogen was passed through the reactor at a rate of 1200 $hr.^{-1}$ GHSV.

## PART B

The catalyst was then tested under epoxidation conditions as shown at Table 1, Runs 1 and 2, using a feed of propylene glycol (LHSV=4.14 $hr.^{-1}$) and a $N_2$ carrier gas flowing at a rate of 1969 $hr.^{-1}$ GHSV. Product samples were removed at the on-stream times (measured from completion of calcination)shown at Table 1, analyzed and the results summarized therein at Runs 1 and 2.

## EXAMPLE 1

A CsOH impregnated silica gel catalyst prepared in accordance with Comparative Example 1, part A, was tested in accordance with part B of Comparative Example 1 with the exception that 5.67 wt.% of the propylene glycol portion of the feed was substituted for water. A mixture of the combined propylene glycol and water feed was then fed to the reactor at an LHSV 4.14 $hr.^{-1}$.

Product samples were removed at the on-stream times shown at Table 1, Runs 3 to 5, analyzed, and the results summarized therein.

## EXAMPLE 2

A CsOH impregnated silica gel catalyst prepared in accordance with Comparative Example 1, part A, was tested in accordance with Comparative Example 1, part B, with the exception that the feed comprised a mixture of MEK, xylene, and propylene glycol, at a weight percent composition as shown at Table 1, Run 6. The feed mixture was passed through the reactor at an LHSV of 12 $hr.^{-1}$. A product sample was removed after an on-stream time of 5.3 hours, analyzed and the results summarized at Run 6.

## EXAMPLE 3

Example 2 was repeated with the exception that the feed also contained water and possessed a composition on a weight percent basis as shown at Table 1, Run 7.

Comparative Example 2

PART A

Silica gel (36.45g) was impregnated with a potassium acetate solution prepared by dissolving 4.37g potassium acetate in 150cc water in a vacuum rotary drier. After rolling silica gel granules in the potassium acetate solution for 40 minutes at room temperature, and then 40 minutes at 52°C, water was removed by applying partial vacuum to the drier at 90°C.

PART B

The dried catalyst was then placed in the reactor and subjected to a pretreatment wherein nitrogen was passed through the reactor (GHSV = 2500 $hr.^{-1}$) at room temperature (i.e., 20 to 25°C) for 18 hours.

PART C

The pretreated catalyst was then tested in accordance with Comparative Example 1, part B, at the reaction conditions shown at Table 1, Run B. Product was removed at 5.3 hours on-stream time, analyzed, and the results summarized at Table 1, Run 8.

EXAMPLE 4

The potassium acetate impregnated silica gel catalyst prepared and pretreated in accordance with Comparative Example 2, parts A and B was tested in accordance with Comparative Example 1, part B, with the exception that a feed mixture of MEK and propylene glycol as described at Table 1, Run 9, was employed in the absence of nitrogen carrier gas at the reaction conditions shown at Table 1, Run 9. Product was removed after 5.3 hours on-stream time, analyzed, and the results summarized at Table 1, Run 9.

Comparative Example 3

PART A

Silica gel (28.85g) was impregnated with a cesium acetate solution prepared by dissolving 6.64g of cesium acetate in 150cc of water in a vacuum rotary drier. After rolling silica gel granules at room temperature for 0.52 hours and then at 57°C for 1.8 hours, water was removed by applying partial vacuum to the drier.

PART B

The dried cesium acetate catalyst was then subjected to the nitrogen pretreatment in accordance with Comparative Example 2, part B.

PART C

The catalyst after pretreatment was then tested at the reaction conditions, and using a feed composition, as shown at Table 1, Run 10. Product was removed at 5.3 hours on-stream time, analyzed, and the results summarized therein.

Comparative Example 4

Comparative Example 3 was repeated with the exception that a nitrogen carrier gas was employed in the feed and the LHSV of the propylene glycol was 4.14 hr.$^{-1}$ rather than 12.0 hr.$^{-1}$. The results of product analysis at 5.3 hours on-stream are shown at Table 1, Run 11.

EXAMPLE 5

Comparative Example 3 was repeated with the exception that 60 weight % of the propylene glycol feed was substituted for MEK. The results of product analysis at 5.3 hours on-stream time are summarized at Table 1, Run 12.

Table 1

| Run No. | Corresponding Ex. or Comp. Ex. No. | Silica Gel Impregnant | Feed Composition (wt.%) MEK | Xylene | P.G. | $H_2O$ | Testing Conditions Feed Rate (LHSV) | $N_2$ Gas (GHSV) | Furnace Temp. (°C) | On-stream Time (hrs.) | Conv. (%) | P.O. Sel. (%) | Yield (%) |
|---|---|---|---|---|---|---|---|---|---|---|---|---|---|
| 1 | C.Ex.1 | CsOH | 0 | 0 | 100 | 0 | 4.14 | 1969 | 360 | 5.3 | 16.5 | 43.2 | 7.1 |
| 2 | C.Ex.1 | CsOH | 0 | 0 | 100 | 0 | 4.14 | 1969 | 360 | 10 | 15.2 | 39.8 | 6.0 |
| 3 | Ex.1 | CsOH | 0 | 0 | 94.33 | 5.67 | 4.14 | 1969 | 360 | 5.3 | 16.5 | 51.6 | 8.5 |
| 4 | Ex.1 | CsOH | 0 | 0 | 94.33 | 5.67 | 4.14 | 1969 | 360 | 10 | 17.5 | 56.2 | 9.8 |
| 5 | Ex.1 | CsOH | 0 | 0 | 94.33 | 5.67 | 4.14 | 1969 | 360 | 50 | 18.7 | 56.2 | 10.5 |
| 6 | Ex.2 | CsOH | 50.15 | 10.03 | 39.82 | 0 | 12.0 | 0 | 360 | 5.3 | 27.8 | 70.5 | 19.6 |
| 7 | Ex.3 | CsOH | 48.22 | 9.64 | 39.78 | 2.36 | 12.0 | 0 | 360 | 5.3 | 27.9 | 85.1 | 23.7 |
| 8 | C.Ex.2 | KAC | 0 | 0 | 100 | 0 | 4.14 | 1969 | 380 | 5.3 | 17.6 | 51.1 | 9.0 |
| 9 | Ex.4 | KAC | 60 | 0 | 40 | 0 | 12.0 | 0 | 380 | 5.3 | 29.8 | 69.7 | 20.8 |
| 10 | C.Ex.3 | CsAc | 0 | 0 | 100 | 0 | 12.0 | 0 | 360 | 5.3 | 12.0 | 48.3 | 5.8 |
| 11 | C.Ex.4 | CsAc | 0 | 0 | 100 | 0 | 4.14 | 1969 | 360 | 5.3 | 16.3 | 46.8 | 7.6 |
| 12 | Ex.5 | CsAc | 60 | 0 | 40 | 0 | 12.0 | 0 | 360 | 5.3 | 27.7 | 76.2 | 21.1 |

Ac = acetate
MEK = methylethylketone
P.G. = propylene glycol
P.O. = propylene oxide

Discussion of Results

Referring to Table 1, Runs 1 and 2, illustrate the performance of the CsOH/silica gel catalyst at 5.3 and 10 hours on-stream time respectively. The selectivity and conversion drop from 43.2% and 16.5% respectively, to 39.8% and 15.2%. In contrast, the inclusion of water in the feed (Runs 3 to 5) results in an increase in conversion over time (e.g., 5.3 to 50 hours on-stream time), while selectivity is stabilized at about 56.2%.

Run 6 illustrates the effect of employing MEK in the absence of water, namely, conversion is 27.8% and selectivity is 70.5%. When water is added to the feed (Run 7), conversion remains the same but an additional increase in selectivity to 85.1% is observed.

Likewise the effect of employing MEK in the feed using a potassium acetate catalyst is illustrated by comparing Runs 8 and 9, namely, both the conversion and selectivity are improved.

Runs 10 and 12 illustrate the same effect of MEK using a cesium acetate catalyst.

In contrast, Run 11 employing only a $N_2$ carrier gas, illustrates a slight improvement in yield relative to Run 10 (no carrier), but the presence of MEK in Run 12, in lieu of nitrogen results in nearly a three fold increase in yield.

Unless otherwise specified, in the following examples and comparative examples summarized at Tables 2 and 3 the reactor effluent for the first 30 minutes after each start-up or charge of the reactor is discarded, but is collected thereafter for a period of 45 minutes in an ice trap. The total liquid effluent collected during this time is analyzed by gas chromatography. Unless otherwise specified, the catalysts tested hereinafter were each calcined in the reactor at a 438°C furnace temperature overnight (i.e. 16 hrs) while dry nitrogen was passed through the reactor at a rate of 1200 $hr^{-1}$ GHSV.

EXAMPLE 6

Silica gel (30.2 g) granules were mixed with 180ml of a RbOH solution prepared by diluting 8.79g of a 50 w/w % solution of RbOH in water with 175ml of water, in a vacuum rotary drier maintained at 62°C with a water bath for 2 hours.

The water was removed by evaporation at 90°C (water bath temperature) by applying vacuum to the rotary drier. After calcination the resulting RbOH impregnated silica gel was tested under epoxidation conditions as shown at Table 2 Runs 13 and 14 and the results summarized therein.

EXAMPLE 7

In accordance with the procedure of Example 6, 33.08g of silica gel were impregnated with a CsOH aqueous solution prepared by dissolving 5.39g CsOH in 195ml water. The catalyst was calcined and tested. The catalyst testing conditions and results are summarized at Table 2, Runs 15 and 16.

EXAMPLE 8

Before impregnating the silica gel, it was immersed in excess water at room temperature for 0.5 hours, and excess water removed by decantation. The resulting hydrogel was then precalcined at 540°C in air for 16 hours. Upon completion of the precalcination, the silica gel was rehydrated with water a second time as described above and again precalcined at 540°C.

A 30.42g sample of the precalcined silica gel was then impregnated with a CsOH aqueous solution containing 6.23g CsOH dissolved in 180ml water in accordance with the procedures of Example 6, and again calcined at 438°C.

The catalyst test conditions and results are summarized at Table 2, Runs 17 and 18.

EXAMPLE 9

A 28.61g sample of silica gel was impregnated with a RbOH aqueous solution containing 3.11g RbOH, and 175ml of water in accordance with Example 6. After calcination, the catalyst was tested and the test conditions and results summarized at Table 2, Runs 19 and 20.

EXAMPLE 10

A 24.66g sample of silica gel was impregnated with an aqueous KOH solution containing 1.62g KOH and 180ml of water in accordance with Example 6. After calcination the catalyst was tested; test conditions and results being summarized at Table 2, Runs 21 and 22.

Comparative Example 5

A sample of $Na_2B_2O_4 \cdot 8H_2O$ was calcined at 430°C for 3 hours in air. The calcined product was ground to -6 + 18 mesh granules (Tyler Series) and further calcined in-situ at 438°C as described hereinabove. A 5cc sample of this catalyst was tested for propylene glycol dehydration. The test conditions and results are listed in Table 3, Runs 28 to 30. Although this catalyst has high activity, the PO selectivity is unattractively low. This type of catalyst is disclosed in Fr. Patent No. 2,345,440 and U.S. Patent No. Re. 29,579.

Comparative Example 6

A 5cc sample of anhydrous $Na_2B_4O_7$ was obtained, calcined and tested; the test conditions and results are summarized at Table 3, Runs 31 and 32. This catalyst is also disclosed in Fr. Patent No. 2,345,440 and U.S. Patent No. Re. 29,579.

Comparative Example 7

A 68.27g sample of alpha-$Al_2O_3$ granules -9 + 20 mesh (Tyler series) was impregnated in accordance with the procedures of Example 6, with a potassium metaborate aqueous

solution prepared by dissolving 3.72g $K_2B_2O_4 \cdot XH_2O$ in 180ml of water. The catalyst was calcined and tested; the test conditions and results being summarized at Table 3, Runs 33 to 35. The catalyst of this comparative example is disclosed in Fr. Patent No. 2,345,440.

Comparative Example 8

A 33.73g sample of silica gel was impregnated with a potassium metaborate aqueous solution prepared by dissolving 33.73g $K_2B_2O_4 \cdot XH_2O$in 150ml of water in accordance with the procedures of Example 6. The catalyst was calcined and tested and the test conditions summarized at Table 3, Runs 36 and 38. Substantial deposition of a heavy unknown material was observed in the cooler part of the reactor effluent line at the exit of the furnace. This comparative example illustrates the performance of a catalyst comprising a weakly acidic support and an alkali metal basic compound.

Comparative Example 9

A 58.98g sample of 3.2mm MgO pellets (obtained from Ventron Company) were impregnated with potassium metaborate aqueous solution prepared by dissolving 3.60g $K_2B_2O_4 \cdot XH_2O$ in 185ml of water. The solution and pellets were placed in a rotary mixer at 62°C for 0.5 hours, and then dried in a rotary drier under partial vacuum. The catalyst was calcined and tested; testing conditions and results being summarized at Table 3, Runs 39 and 40. This comparative example illustrates the performance of catalyst composition comprising basic support and an alkali metal compound.

Comparative Example 10

A 57.48g sample of gamma-$Al_2O_3$, 3.2mm, pellets (available from Ventron Company) having a surface area of $100m^2/g$ was impregnated with an aqueous KOH solution prepared by dissolving 3.25g KOH in 190ml water. The impregnation was carried out by rolling the gamma-$Al_2O_3$ pellets at 62°C (water

bath temp.) for 0.8 hours in a vacuum rotary drier. Water was evaporated under partial vacuum at about 90°C (water bath temp.). The dried catalyst was calcined and tested. The catalyst testing conditions and results are summarized at Table 3, Run 41. This comparative example illustrates the performance of catalyst composition comprising neutral or basic support and an alkali metal hydroxide similar to that disclosed in Fr. Patent No. 2,345,440. Gamma-$Al_2O_3$ is an acidic material.

Comparative Example 11

An 82.31g sample of MgO pellets, as used in Comparative Example 9, was impregnated with an aqueous KOH solution prepared by dissolving 2.89g KOH in 180ml water in accordance with the procedure of Comparative Example 10. The catalyst was calcined and tested. Catalyst testing conditions and results are summarized at Table 3, Run 42.

This comparative example illustrates the performance of a catalyst comprising a basic support and alkali metal hydroxide.

Comparative Example 12

A lithium hydroxide solution was prepared by dissolving a 0.60g LiOH in 175cc of water. Silica gel granules (29.6g) were impregnated with above solution, by rolling the granules at 47°C in the lithium hydroxide solution for an hour. Water was then removed from the solution with a rotary drier at 90°C by applying a partial vacuum. The catalyst was then calcined and tested, with the test results summarized at Table 3, Runs 43 and 44 at two different feed rates. This catalyst shows unsatisfactory results compared to those of runs 1-10 and 23-27.

Comparative Example 13

A rubidium hydroxide solution was prepared by mixing 9.84g of an aqueous 50 wt.% RbOH solution with 100ml

of water. $GeO_2$ powder (93.42g) was impregnated with the above solution by heating the same to a temperature of about 100°C for a time sufficient to obtain a thick wet paste. The paste was dried in a vacuum oven at 122°C for 4 hours. The dried paste was ground to -6 + 6 mesh (Tyler Series) granules. The granules were calcined at 900°C for 4.5 hours in air and tested. The test results of this catalyst are summarized at Table 3, Run 45.

EXAMPLE 11

A silica gel sample (36.45g) as employed in Example 9 was impregnated with a potassium acetate solution prepared by dissolving 4.37g anhydrous potassium acetate in 150cc of water. The impregnation was carried out by rolling the silica gel granules in a vacuum rotary drier at room temperature for 40 minutes and then at a 62°C water bath temperature for 40 minutes, followed by evaporation of the water at about 90°C water bath temperature under partial vacuum.

About 5cc of catalyst sample was placed in the reactor and heated under nitrogen to 400°C at which time the feed was introduced. The test results are summarized at Table 2, Runs 23 and 24. This type of catalyst is described in U.S. Patent No. 4,226,780.

EXAMPLE 12

The silica gel (28.85g) as employed in Example 9 was impregnated with a cesium acetate solution prepared by dissolving 6.64g of anhydrous cesium acetate in 150cc water. The impregnation was carried out by rolling the silica gel granules with the cesium acetate solution in a vacuum rotary drier at room temperature for 0.52 hours and then at 62°C water bath temperature for 1.82 hours followed by evaporating water at about 90°C water bath temperature under partial vacuum. The catalyst (5cc) was tested in accordance with Example 11. The test results are summarized at Table 2, Runs 25 to 27.

Table 2

| Run No. | Corresponding Ex. No. | Silica Gel Impregnant | CATALYST TESTING CONDITIONS: Reactant** Feed | Furnace Temp. (°C) | Feed Rate (LHSV) | PG Conversion (X) | % SELECTIVITY TO: PO | PA | Ac | AA | DPG | PO Yield (%) |
|---|---|---|---|---|---|---|---|---|---|---|---|---|
| 13 | 6 | RbOH | A | 400 | 11 | 45.2 | 71.0 | 11.1 | 6.8 | 4.8 | 4.5 | 32.1 |
| 14 | 6 | RbOH | A | 380 | 11 | 32.7 | 72.4 | 8.6 | 6.4 | 4.3 | 3.0 | 23.7 |
| 15 | 7 | CsOH | A | 400 | 11 | 56.5 | 67.5 | 11.4 | 6.3 | 5.5 | 5.2 | 38.1 |
| 16 | 7 | CsOH | A | 380 | 11 | 38.7 | 74.2 | 8.1 | 5.1 | 3.2 | 5.6 | 28.7 |
| 17 | 8 | CsOH* | A | 400 | 11 | 45.2 | 63.6 | 8.9 | 5.2 | 4.5 | 6.3 | 31.3 |
| 18 | 8 | CsOH* | A | 375 | 11 | 27.8 | 75.2 | 7.3 | 5.3 | 3.8 | 8.0 | 20.9 |
| 19 | 9 | RbOH | A | 400 | 11 | 47.2 | 64.1 | 10.1 | 5.6 | 3.3 | 6.3 | 30.3 |
| 20 | 9 | RbOH | A | 400 | 7 | 57.1 | 62.2 | 11.3 | 6.4 | 4.1 | 5.3 | 35.5 |
| 21 | 10 | KOH | A | 400 | 11 | 35.0 | 69.6 | 13.3 | 8.4 | 3.8 | 7.9 | 24.4 |
| 22 | 10 | KOH | A | 400 | 6 | 49.2 | 64.0 | 14.3 | 8.9 | 4.7 | 5.7 | 31.5 |

* Precalcined at 540°C

** A = 40 wt.% propylene glycol (PG) dissolved in methylethylketone (MEK)

PO = propylene oxide
PA = propionaldehyde
Ac = acetone
AA = allyl alcohol
DPG = dipropyleneglycol

Table 2

| Run No. | Corres-ponding Ex. No. | Silica Gel Impregnant | CATALYST TESTING CONDITIONS Reactant Feed | Furnace Temp. (°C) | Feed Rate (LHSV) | PG Conver-sion (%) | % SELECTIVITY TO PO | PA | Ac | AA | DPG | PO Yield (%) |
|---|---|---|---|---|---|---|---|---|---|---|---|---|
| 23 | 11 | K Act | A | 400 | 11 | 47.5 | 42.3 | 23.2 | 11.0 | 69 | 1.1 | 20.0 |
| 24 | 11 | K Act | A | 400 | 6 | 52.6 | 40.5 | 25.5 | 11.1 | 6.3 | 1.0 | 21.3 |
| 25 | 12 | Cs Act | A | 400 | 11 | 63.3 | 36.7 | 28.4 | 9.3 | 8.3 | 0.6 | 23.2 |
| 26 | 12 | Cs Act | A | 375 | 11 | 43.0 | 51.4 | 21.0 | 8.5 | 9.5 | 1.3 | 22.1 |
| 27 | 12 | Cs Act | A | 360 | 12 | 35.9 | 65.0 | 13.1 | 5.3 | 6.6 | 6.2 | 23.3 |

* Precalcined at 540°C

** A = 40 wt.% propylene glycol (PG) dissolved in methylethylketone (MEK)

PO = propylene oxide
PA = propionaldehyde
Ac = acetone
AA = allyl alcohol
DPG = dipropyleneglycol
Act = acetate

Table 3

| Run No. | Corres-ponding Ex. No. | Catalyst | CATALYST TESTING CONDITIONS Reactant** Feed | Furnace Temp. (°C) | Feed Rate (LHSV) | PG Conver-sion (%) | % SELECTIVITY TO PO | PA | Ac | AA | DPG | PO Yield (%) |
|---|---|---|---|---|---|---|---|---|---|---|---|---|
| 28 | 5 | $Na_2B_2O_4$ | A | 400 | 11 | 76.7 | 21.8 | 6.5 | 5.9 | 0.4 | ND | 16.7 |
| 29 | 5 | " | A | 400 | 14 | 74.8 | 20.0 | 7.0 | 6.4 | 0.3 | ND | 15.0 |
| 30 | 5 | " | A | 380 | 14 | 58.0 | 18.1 | 5.8 | 5.8 | 0 | ND | 10.5 |
| 31 | 6 | $Na_2B_4O_7$ | A | 400 | 11 | 83.5 | 15.6 | 5.1 | 4.7 | 0 | ND | 13.0 |
| 32 | 6 | " | A | 400 | 15 | 66.8 | 16.7 | 5.4 | 5.0 | 0 | ND | 11.1 |
| 33 | 7 | alpha-$Al_2O_3$ + $K_2B_2O_4$ | A | 400 | 11 | 80.0 | 11.6 | 8.7 | 8.4 | 0 | ND | 9.3 |
| 34 | 7 | " | A | 400 | 15 | 68.0 | 14.0 | 9.2 | 9.3 | 0 | ND | 9.5 |
| 35 | 7 | " | A | 380 | 15 | 43.6 | 21.8 | 7.8 | 6.9 | 0 | ND | 9.5 |
| 36 | 8 | silica gel + $K_2B_2O_4$ | A | 400 | 11 | 35.5 | 60.6 | 11.3 | 6.8 | ND | 4.0 | 21.5 |
| 37 | 8 | " | A | 400 | 15 | 38.4 | 53.6 | 8.7 | 5.7 | ND | 4.1 | 20.6 |
| 38 | 8 | " | A | 400 | 6 | 50.2 | 55.6 | 12.4 | 7.5 | ND | 4.0 | 27.9 |
| 39 | 9 | MgO + $K_2B_2O_4$ | A | 400 | 11 | 63.3 | 11.4 | 5.7 | 7.8 | 4.8 | 1.0 | 7.2 |

Table 3

| Run No. | Corresponding Ex. No. | Catalyst | CATALYST TESTING CONDITIONS: Reactant** Feed | Furnace Temp. (°C) | Feed Rate (LHSV) | PG Conversion (%) | % SELECTIVITY TO: PO | PA | Ac | AA | DPG | PO Yield (%) |
|---|---|---|---|---|---|---|---|---|---|---|---|---|
| 40 | 9 | " | A | 380 | 11 | 44.7 | 15.6 | 6.2 | 8.9 | 4.1 | 2.9 | 7.0 |
| 41 | 10 | $-Al_2O_3$ + KOH | A | 400 | 11 | 66.8 | 7.9 | 10.2 | 8.9 | 6.6 | 4.8 | 5.3 |
| 42 | 11 | MgO + KOH | A | 400 | 11 | 47.0 | 8.8 | 5.4 | 6.5 | 6.1 | 0.7 | 4.1 |
| 43 | 12 | LiOH/Silica gel | A | 400 | 11 | 20.3 | 14.0 | 39.3 | 14.9 | ND | ND | 2.8 |
| 44 | 12 | " | A | 400 | 6 | 23.8 | 18.4 | 36.3 | 14.0 | ND | ND | 4.4 |
| 45 | 13 | $RbOH/GeO_2$ | A | 400 | 11 | 84.8 | 0.5 | 1.6 | 4.6 | 0 | 4.0 | 0.4 |

** See Table 2

Discussion of Results

As may be seen from the data of Table 2, propylene oxide selectivities range from 62.2 to 75.2% while propylene oxide yields vary from 20.9 to 38.1%.

In contrast the alkali metal salts and non-silica gel supported alkali metal salts show very poor PO selectivity and yields. When the alkali metal borate salt is supported on a silica gel, PO selectivities increase (see Runs 36 to 38) relative to the unsupported form, but at similar reaction temperatures the PO selectivities and yields of Runs 36 to 38 are still inferior to those of the present invention. Similar results are observed for runs 39 to 42.

Referring to Runs 43 and 44, it can be seen that a silica gel supported LiOH catalyst results in significantly reduced conversions and particularly propylene oxide selectivities relative to the runs of Table 2 thereby indicating that Li is an unsuitable alkali metal for use in the present invention.

Referring to Run 45 it can be seen that $RbOH/GeO_2$ catalyst wherein the germanium thereof is immediately below silicon in the periodic chart, is ineffective in producing PO, thereby indicating that the silica support of the present invention is not inert and contributes to the performance of the catalyst composition.

Referring to Runs 23 and 24 it can be seen that a potassium acetate/silica gel catalyst composition, is quite active in terms of conversion, but results in a drop in PO selectivity relative to Runs 2 and 22.

Comparing runs 25 and 26 (cesium acetate) with Runs 15 and 16 it can be seen that at similar reaction temperatures, propylene oxide selectivity is reduced for the acetate although the conversions are higher for Runs 25 and 26. However, such higher conversions produce more propionaldehyde by-product. It is concluded that the hydroxide is preferred over the acetate.

CLAIMS:

1. A process for expoxidizing at least one organic polyol compound containing at least one pair of vicinal hydroxy groups which comprises:

(A) providing a feed consisting essentially of a mixture of said polyol compound and at least one promoter selected from the group consisting of water, aldehyde or ketone; said promotor having an identity and being present in an amount sufficient to increase at least one of the conversion of said polyol and selectivity to epoxide, relative to the absence of said promoter;

(B) contacting said feed with a catalyst composition comprising a solid acidic amorphous silica having adsorbed thereon, at least one member selected from the group consisting of alkali metal hydroxide, an alkali metal oxide, and alkali metal carboxylate, said alkali metal being selected from at least one member of the group consisting of K, Rb, and Cs, said contacting being conducted in a manner and under conditions sufficient to convert at least one pair of said vicinal hydroxy groups to its corresponding epoxide group.

2. The process of Claim 1 wherein said promoter is selected from the group consisting of water, methylethylketone, and mixtures thereof.

3. The process of Claim 1 or 2 wherein said catalyst composition the amorphous silica is a silica gel.

4. The process of Claim 3 wherein in said catalyst composition the silica gel has adsorbed thereon at least one member selected from the group consisting of cesium hydroxide, potassium acetate, cesium acetate and mixtures thereof.

5. The process of any one of the preceding claims wherein said aldehyde or ketone containing promoter is represented by the structural formula:

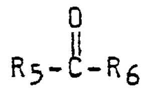

wherein $R_5$ is selected from the group consisting of $C_1$ to $C_{10}$ alkyl, $C_2$ to $C_{10}$ alkenyl, $C_6$ to $C_{10}$ aryl, and aralkyl and alkaryl wherein the alkyl and aryl portions thereof are as described above, said alkyl, alkenyl or alkyl portions of $R_5$ being optionally substituted with an oxo group; and $R_6$ is selected from the group consisting of hydrogen, alkyl, alkenyl, aryl, aralkyl, and alkaryl as described in conjunction with $R_5$; said $R_5$ and $R_6$ when both representing a hydrocarbyl group together can constitute a $C_4$ to $C_{12}$ cycloalkylene group, said cycloalkylene group being also optionally substituted with an oxo group.

6. The process of any one of the preceding claims wherein the promoter is at least one ketone and comprises from 1 to 95%, by weight, of the feed, based on the combined weight of vicinal diol and promoter.

7. The process of any one of claims 1-4 wherein the promoter is water and comprises from 0.1 to 25%, by weight, of the feed, based on the combined weight of vicinal diol and promoter.

8. The process of any one of claims 1-5 wherein the feed comprises in admixture, a weight% ratio of vicinal diol, water, and ketone of from 50:1:1000 to 3:3:1.

9. The process of Claim 8 wherein the ketone is methylethylketone.

10. The process of any one of the preceding claims wherein said contacting is conducted in the absence of a carboxylic acid.

11. The process of any one of the preceding claims wherein the alkali metal is selected from the group consisting of rubidium and cesium.

12. The process of any one of the preceding claims wherein said polyol is an alkane vicinal diol having from 2 to 20 carbon atoms.

13. The process of Claim 12 wherein said polyol is selected from the group consisting of ethylene glycol, propylene glycol and butane-1,2-diol.

14. The process of any one of the preceding claims wherein said alkali metal hydroxide is chemically adsorbed on the silica gel support by reaction of the surface hydroxyl groups present thereon with said alkali metal hydroxide.

15. The process of any one of the preceding claims wherein said catalyst composition is calcined at a temperature of from 200 to 950$^{\circ}$C for a period of from 0.1 to 40 hours.

16. The process of any one of the preceding claims wherein said contacting is conducted in the vapor phase.

European Patent Office

# EUROPEAN SEARCH REPORT

0145447

Application number

EP 84308450.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US - A - 4 297 287 (COSTANTINI)<br>* Claim 1 *<br>-- | 1 | C 07 D 301/02 |
| D,A | US - A - 4 226 780 (FOUQUET)<br>* Claims 1,2 *<br>-- | 1,3-5 | |
| P,A | EP - A1 - 0 122 025 (ATLANTIC RICHFIELD COMP.)<br>* Claims 1-4 *<br>-- | 1,3-5 | |
| P,A | EP - A1 - 0 122 026 (ATLANTIC RICHFIELD COMPANY)<br>* Claims 1-4 *<br>---- | 1,3-5 | |

| TECHNICAL FIELDS SEARCHED (Int. Cl.4) |
|---|
| C 07 D 301/00 |

The present search report has been drawn up for all claims

| Place of search | Date of completion of the search | Examiner |
|---|---|---|
| VIENNA | 06-03-1985 | BRUS |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82